# EUROPEAN PATENT SPECIFICATION

(11) **EP 4 088 701 B1**
(45) Date of publication and mention of the grant of the patent: **07.08.2024**
(21) Application number: 21173212.8
(22) Date of filing: 11.05.2021
(51) Int. Cl.: A61F 13/26

(54) **TAMPON APPLICATOR**
TAMPONAPPLIKATOR
APPLICATEUR DE TAMPON

(43) Date of publication of application: 16.11.2022
(60) Divisional application: 24184091.7
(73) Proprietor: Ontex BV, 9255 Buggenhout (BE); Ontex Group NV, 9320 Erembodegem (BE)
(72) Inventor: JANSEN, Bart, 2640 Mortsel (BE); LESAGE, Henri, 9900 Eeklo (BE); SEQUEIRA NOGUEIRA, Frederico Álvaro, 02625 Bautzen (DE)
(74) Representative: Saurat, Thibault

(56) References cited:
- WO-A2-2007/146270
- WO-A2-96/40031
- US-A1- 2002 183 182
- US-A1- 2003 163 080

## Description

### TECHNICAL FIELD

The invention pertains to the technical field of cardboard or other paper-based tampon applicators as well as a method to manufacture such tampon applicator.

### BACKGROUND

Tampon applicators are generally either a moulded thermoplastic material, such as plastic, or a cardboard or other paper-based materials, such as paperboard or paper laminate, collectively referred to herein as "cardboard".

Moulded plastic is often used to form a tampon applicator. Such applicators have a generally cylindrical shape with a lower expulsion force needed to eject the tampon pledget from the applicator. Furthermore, these moulded plastic applicators can be formed with a high degree of surface smoothness, which results in increased comfort during insertion of the tampon. However, these plastic tampon applicators are usually made from non-biodegradable and non-water dispersible material and thus are not environmentally friendly unless certain expensive plastics are used.

To that end, a variety of commercial tampon applicators appeared on the market which are formed from cardboard. However, these cardboard tampon applicators are formed of two telescoping tubes and can be perceived by consumers as difficult and uncomfortable to insert. Furthermore, these cardboard tampon applicators also lack good griping features and are usually slippery.

Solutions have suggested in the past where the cardboard is coated with a plastic layer, for example publications such as US 5,931,803 or US 6,095,999 describe such solution. Although these disclosures describe an improvement of the opening of the petals and a comfort of use, there is still a need to improve the environmental impact of these applicator and offer a solution that involves less plastic without degrading the performance of the applicator.

To that end, the invention aims to provide a tampon applicator that meets the standard performances of a plastic tampon applicator while maintaining a low environmental impact.

### SUMMARY OF THE INVENTION

The present invention concerns a tampon applicator wherein the tampon applicator comprises cellulose pulp and discrete areas of polymeric material.

The term "discrete" used herein means separate or distinct. In other words, the term "discrete areas of polymeric material" used herein refers to the areas of polymeric material being clearly separated or different in shape or form. In other words, the areas of polymeric material consist of distinct, non-continuous or unconnected elements.

The cellulose pulp constitutes the majority of the composition lowering the environmental impact and the different areas of polymers occupy pre-defined areas and specific function that cardboard material is not fit to occupy. The fact that the polymeric material is present in discrete or individual areas and not coated all over the cardboard enables to further lower the environmental impact of the tampon applicator without affecting its performances.

According to an embodiment, the tampon applicator comprises between about 75 % to about 97 % in weight of cellulose pulp and between about 3 % to about 25 % in weight of polymeric material. The tampon applicator can comprise other additives for example and not limited to pigments, antioxidants, stabilizers, surfactants, waxes, flow promoters, solvents, particulates and materials added to enhance processability of the composition.

According to an embodiment, the polymeric material consists essentially of a bioplastic derived from biomass and/or a biodegradable polymeric material and/or a compostable polymeric material and/or a water-soluble polymeric material and/or a marine biodegradable polymeric material. Such material can be selected from and not limited to the followings: polyhydroxy butyrate (PHB), thermoplastic starch (TPS), polyvinyl alcohol (PVOH), polylactic acid (PLA), polyethylene oxide (PEO), Arboblend^{®}, polyhydroxylakanoate (PHA), polycaprolactone (PCL) and bio-polyethylene (bio-PE), namely low-density polyethylene (LDPE). The tampon applicator can comprise other additives for example and not limited to pigments, antioxidants or pro-oxidant, stabilizers, surfactants, waxes, flow promoters, solvents, particulates and materials added to enhance processability of the composition.

According to an embodiment, the tampon applicator comprises:
- a barrel defining an interior cavity,
- a plunger arranged to slide within the barrel,
wherein the barrel comprises cellulose pulp and discrete areas of polymeric material.

According to an embodiment, the tampon applicator comprising:
- a barrel defining an interior cavity,
- a plunger arranged to slide within the barrel,
wherein the plunger comprises cellulose pulp and discrete areas of polymeric material.

According to an embodiment, the barrel extending in a longitudinal direction L further comprises:
- a discharge end arranged at a first longitudinal end, or distal end, of the barrel and comprising flexible petals,
- a finger grip area arranged at the opposite longitudinal end, or proximal end, of the barrel,
- a main body section arranged between the discharge end and the finger grip area,
wherein the petals and the finger grip area consist essentially of polymeric material or a combination of cellulose pulp and polymeric material and the main body section consists essentially of cellulose pulp.

According to an embodiment, the barrel further comprising at its distal end, tampon pledget retaining means comprising a plurality of protrusions extending inward in the interior of the barrel, wherein the retaining means consist essentially of polymeric material.

According to an embodiment, the plunger comprises at least one finger at its distal end, wherein the at least one finger consists essentially of polymeric material.

According to an embodiment, the barrel comprises at its proximal end a circumferential flange extending outward and/or the plunger comprises at its proximal end a retention flange extending outward, the circumferential flange and/or retention flange consisting essentially of polymeric material.

According to an embodiment, the barrel further comprising at its proximal end a tampon pledget retaining mean comprising a resilient plate extending in the interior of the barrel, the plunger comprising a slit extending in the longitudinal direction of the plunger, the resilient plate being arranged within the slit, wherein the resilient plate consists essentially of polymeric material and/or cellulose pulp.

According to an embodiment, the barrel and/or plunger comprise at least two discrete areas comprising polymeric material, the polymeric material in first area being different from the polymeric material in another area.

According to an embodiment, the plunger comprises an inner sleeve and an outer sleeve with interlocking means, said inner sleeve and/or outer sleeve comprising at least one discrete area of polymeric material.

The invention also pertains to a process, more precisely an injection moulding process, for manufacturing a tampon applicator as described above, the process comprising the following steps:
- providing a cardboard shell comprising at least the main body section of the barrel or the main body section of the plunger;
- injecting the polymeric material in the targeted pre-defined areas;
- removing the tampon applicator.

According to an embodiment, the process comprises the following additional steps:
- punching holes in the carboard shell,
- injecting polymeric material in said holes.

All these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1** illustrates a cross-sectional schematic view of the tampon applicator according to the invention in a compacted configuration.
**FIG. 2** illustrates a cross-sectional schematic view of the tampon applicator in **FIG.1** in a deployed configuration.
**FIG. 3** illustrates an isometric schematic partial cut of a component of the tampon applicator, specifically the barrel.
**FIG. 4** illustrates a cross-sectional schematic view of a component of the tampon applicator, specifically the plunger.
**FIG. 5** illustrates an isometric view of the tampon applicator in a disassembled state according to an embodiment of the invention.
**FIG. 6** illustrates a cross-sectional schematic view of the tampon applicator in a compacted configuration according to another embodiment.

### DETAILED DESCRIPTION OF THE INVENTION

The present invention concerns a tampon applicator. The invention also concerns a tampon assembly comprising said tampon applicator as well as the process for making such tampon applicator.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints except where otherwise explicitly stated by disclaimer and the like.

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

The terms "water-dispersible" or "water-soluble" are equivalent and used herein refer to a material that loses its integrity over time in the presence of water, yet also has a high enough melt flow index and a low enough melt viscosity such that it can be moulded into an article such as a tampon applicator. In other words, the terms "water-dispersible" or "water-soluble" used herein refer to a material that readily breaks apart in unrecognizable pieces upon contact with water as a result of dissolution, solubilization, dissipation, agitation, softening, or any other chemical or mechanical dispersion means.

The term "marine degradable" used herein means a material that has the ability to completely biodegrade under marine environmental conditions including aerobic marine waters or anaerobic marine sediments within a specified timeframe, leaving no toxic substances or residue.

As used herein, the terms "inward" or "inwardly" and "outward" or "outwardly" are in reference to the generally cylindrical shape of the tampon applicator according to the invention. The main components of a tampon applicator, which are the barrel and the plunger, are substantially cylindrical defining with their respective wall of given circumference an interior space (inside), or cavity, and an exterior space (outside). "Inward" and "inwardly" refer to an element extending at least partially radially towards the centre of the cylindrical shape of said component whereas "outward" and "outwardly" refer to an element extending at least partially radially away from the centre of the cylindrical shape of said component. In other words, "inward" means directed toward the interior and situated inside the cylindrical shape whereas "outward" means directed toward the outside or away from a centre and situated outside the cylindrical shape.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "finished" or "final", when used with reference to a product, means that the product has been suitably manufactured for its intended purpose.

"Texture" as used herein refers to the structure, feel, and appearance of an element.

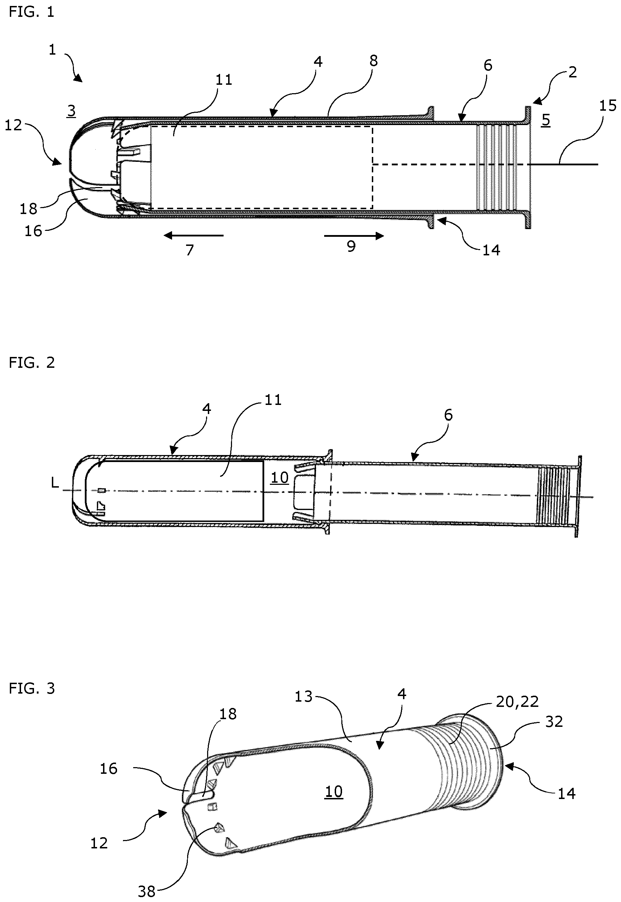

As used herein, the term "proximal end" refers to those portions of the tampon applicator and of its parts that are most remote from the body of a user when a tampon is being emplaced in a body cavity using said applicator. The term "distal end" refers to those portions of the tampon applicator and of its parts that are closest to the body of the user when the tampon is being emplaced. Accordingly, the terms "proximal" or "proximally", and "distal" or "distally", as used herein, specify that a given portion or structure of the tampon applicator or of its parts is relatively closer to, respectively, the proximal end or the distal end of the applicator or of its parts. Similarly, the terms "proximal direction" and "distal direction" refer to the directions towards the proximal end or towards the distal end of the tampon applicator or of its parts, respectively. In FIG. 1, the distal and proximal ends of the tampon applicator 2 and its components, e.g. the barrel 4 and the plunger 6, as meant herein are denoted by reference signs 3 and 5 respectively; and the distal and proximal directions as meant herein are denoted by reference arrows 7 and 9, respectively.

Embodiments according to the disclosure will now be described. It is understood that technical features described in one or more embodiments maybe combined with one or more other embodiments without departing from the intention of the disclosure and without generalization therefrom.

FIG. 1 and FIG. 2 illustrate a cross-sectional view of a tampon assembly 1 comprising a tampon pledget 11 and a tampon applicator 2 according to the invention in a compacted, or non-deployed, configuration (FIG. 1) or in a deployed configuration (FIG. 2). The tampon applicator 2 comprises a barrel 4, also known as the outer tube, and a plunger 6, also known as the inner tube or ejector tube. Both components, i.e. barrel 4 and plunger 6, have a generally cylindrical shape. The plunger 6 has a smaller diameter than the barrel 4 and is arranged in way that it can slide within the barrel 4. In other words, the outer tube 4 is dimensioned to fit closely and telescopically over said plunger 6. The plunger 6 is dimensioned and arranged to easily slide within the barrel 4, with minimal clearance in between. The plunger 6 is also preferably slightly longer that the barrel 4 to ensure complete ejection and proper depth placement of the tampon pledget 11. Being hollow, the plunger 6 also permits proper placement of a withdrawal string 15 such as commonly provided at the withdrawal end, or proximal end, of tampon pledgets 11.

As explained above, the barrel 4 and the plunger 6 have a substantially cylindrical shape. The barrel 4 is hollow thus defining with its housing, or wall 8, an interior cavity 10 configured to store or contain the plunger 6 and the tampon pledget 11. The barrel 4 and the plunger 6 both extend in a longitudinal direction L (illustrated in figure 2). The plunger 6 is also hollow and can define an interior space configured to store or contain the tampon pledget 11 while in the compact configuration. As illustrated in FIG.1, the tampon assembly 1 comprises a group of components arranged to fit closely one within another, we can also say that the barrel 4, pledget 6 and tampon pledget 11 are nested altogether.

The elongated barrel 4 has a discharge end 12 arranged at a first longitudinal extremity, more specifically at the distal end 3 of the tampon applicator 2, and an open end 14 at the opposite longitudinal extremity, i.e. at the proximal end 5 of the tampon applicator 2, or in other words at the extremity opposite to the discharge end 12. The open end 14 has an opening to enable the introduction of the tampon pledget 11 as well as the plunger 6 during the process to manufacture the tampon assembly 1. The barrel 4 comprises a main body section 13 arranged between the discharge end 12 and the open end 14. The plunger 6 is adapted to abut against the tampon pledget's 11 proximal end and push it to expel said tampon pledget 11 through the discharge end 12 of the barrel 4.

As illustrated in FIG. 3, the distal discharge end 12 of the barrel 4 is formed with a plurality, e.g., 2 to 16, more commonly 2 to 8, and most commonly 4 conventional petals 16 which are separated from each other by respective slots 18 or gap. The petals 16 are made relatively flexible and are normally biased in a substantially arcuate closed configuration to form a rounded, or dome-shaped, tip having a central opening at the distal end 3 of the tampon applicator 2. The rounded shape of the distal end 3 helps facilitate the insertion of the tampon applicator 2 into a cavity. The petals 16 are flexible and can be deformed by the tampon pledget 11 when it is being pushed by the plunger 6 and expelled through the discharge end 12 of the barrel 4.

The outer proximal surface of the outer tube 4 may be provided with a finger grip area 20 comprising a ridged or knurled surface, which provides for an anti-slip feature and a firmer grip when this portion of the barrel 4 is being held between fingers. In other words, the finger grip area 20 arranged at the proximal end of the barrel 4 comprises a plurality of deformations 22 extending outwardly and/or inwardly, such as ribs, grooves, indentations, dots or any polygonal shape, to provide a texture to the proximal end of the barrel 4. As illustrated in FIG. 3, the deformations 22 comprise circumferential rings extending radially outward, i.e. away from the interior cavity 10. The deformations 22 can be arranged in rows, parallel at uniform distant from one another. In the case of dots or any other polygonal shapes, the deformations 22 can be arranged uniformly or in a staggered configuration. The barrel 4 may further comprise an outwardly directed circumferential flange 32 at its proximal end.

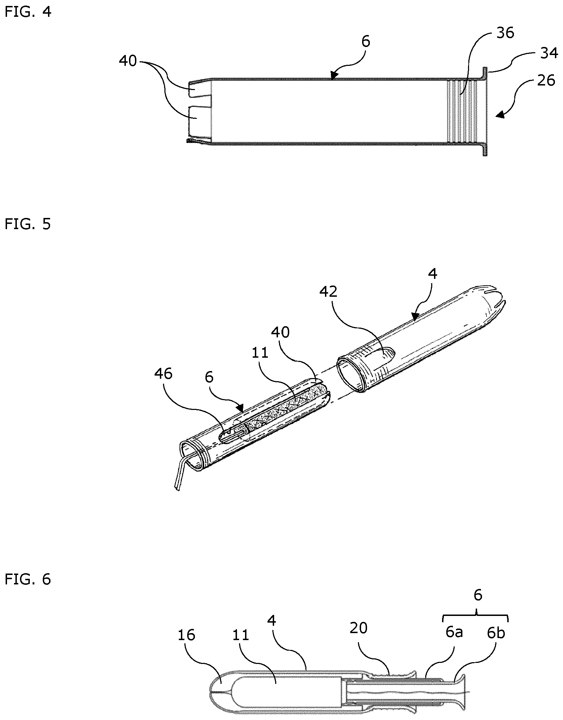

As illustrated in FIG. 4, the plunger 6 may also include an outwardly directed circumferential retention flange 34 at its proximal end. The retention flange 34 controls the extent of insertion of the plunger 6 into the barrel 4 of the tampon applicator 2. The retention flange 34 of the plunger 6 can abut against the proximal end of the barrel 4 and/or the flange 32 of the barrel 4 when the ejector tube 5 is fully inserted into the outer tube 4. The inner, or outer, proximal portion of the plunger 6 may optionally be provided with a ridged or knurled surface 36, which provides for a firmer grip and anti-slip feature for a finger inserted into, or positioned onto, the plunger 6 tube when pushing the plunger 6, said ridged surface 36 can also provide rigidity to the plunger 6.

The tampon applicator 2 according to the invention comprises cellulose pulp and discrete areas of polymeric material. In other words, the tampon applicator 2 is made out essentially of, or consists essentially of cellulose pulp, meaning cardboard or other paper-based materials, with at least two distinct and separate elements of said tampon applicator 2 comprising polymeric material exclusively or a combination of cellulose pulp and polymeric material, with eventually traces of additional materials which do not significantly affect the desired characteristics of a given composition or product as listed previously. These elements are described hereunder.

More specifically, the discrete and/or separate areas of polymeric material are arranged to ensure specific and pre-defined functions of the tampon applicator and thus the good expulsion of the tampon pledget 11 from said applicator 2. In other words, the discrete elements of the tampon applicator 2 that are consisting essentially of polymeric material exclusively or a combination of cellulose pulp and polymeric material correspond to features of the tampon applicator 2. They can be for example the petals 16 and the finger grip area 20. The petals 16 can for example consist essentially of polymeric material exclusively or the petals 16 can consist of a layer of cardboard and a layer of polymeric material that was overmoulded onto the cardboard layer. The finger grip area 20 can consist essentially of polymeric material exclusively or the finger grip area 20 can consist of a cardboard layer with a plurality of rings of polymeric material being overmoulded onto the circumference of the cardboard layer. For example will

As explained above, the tampon applicator 2 according to the invention comprises different areas that consists essentially of cellulose pulp, polymeric material or a combination of cellulose pulp and polymeric material.

In order to limit the environmental impact of the tampon applicator 2, said tampon applicator 2 comprises between about 75 % to about 97 % in weight of cellulose pulp and between about 3 % to about 25% in weight of polymeric material. The tampon applicator 2 can comprise other additives for example and not limited to pigments, antioxidants, stabilizers, surfactants, waxes, flow promoters, solvents, particulates and materials added to enhance processability of the composition. The total weight of the entire tampon applicator 2 comprises between about 75 % to about 97 % in weight of cellulose pulp and between about 3 % to about 25 % in weight of polymeric material. For example, the total weight of the entire tampon applicator can comprise 90 wt. % of cardboard, 7 wt. % of polymeric material and 3 wt. % of additives. In other words, the cellulose derivative can constitute from about 75 wt. % to about 97 wt. % of the composition based on the total weight of the composition of the tampon applicator 2 and the polymeric material can constitute from about 3 wt. % to about 25 wt. % of the total weight of the composition of the tampon applicator 2. The tampon applicator 2 of the present invention comprises a total of from about 3 % to about 25 %, more preferably from 5 % to 15 % of polymeric material by weight of the applicator. The tampon applicator 2 of the present invention comprises a total of from about 75 % to about 97 %, more preferably from 80 % to 95 % of cellulose pulp by weight of the applicator.

According to an aspect of the invention, the polymeric material may also have a limited environmental impact. To that end, the polymeric material consists essentially of a polymeric material derived from biomass and/or a biodegradable polymeric material and/or a compostable polymeric material and/or a water-soluble polymeric material and/or a marine biodegradable polymeric material. Such material can be selected from, and not limited to, the followings: polyhydroxy butyrate (PHB), thermoplastic starch (TPS), polyvinyl alcohol (PVOH), polylactic acid (PLA), polyethylene oxide (PEO), Arboblend^{®}, polyhydroxylakanoate (PHA), polycaprolactone (PCL) and bio-polyethylene (bio-PE), namely low-density polyethylene (LDPE). The tampon applicator can comprise other additives for example and not limited to pigments, antioxidants or pro-oxidant, stabilizers, surfactants, waxes, flow promoters, solvents, particulates and materials added to enhance processability of the composition.

The polymeric material can also be water-soluble or water-dispersible. Given that cardboard is also water-dispersible, a tampon applicator comprising both material would be of limited impact on the environment.

The polymeric material can also be marine degradable. Given that cardboard is also marine degradable, a tampon applicator comprising both material would be of limited impact on the environment.

The polymeric material can present several of the properties cited above. In other words, the polymeric material can be a bioplastic derived from biomass and/or a biodegradable polymeric material and/or a compostable polymeric material and/or a water-soluble polymeric material and/or a marine biodegradable polymeric material.

For example, PVOH and PEO both correspond to a material that is biodegradable and water-soluble. TPS and PHA are both marine biodegradable bioplastics derived from biomass. PLA and PHB are biodegradable plastics and bio-based plastics. It is also possible to have a mixture of different polymers. PCL is a biodegradable plastic.

As explained above, the tampon applicator 2 comprises a plunger 6 and a barrel 4 comprising an open end 14 configured to receive said plunger 6. According to an embodiment, the plunger 6 consists essentially of cellulose pulp and the barrel 4 comprises cellulose pulp and discrete areas of polymeric material. This embodiment is particularly interesting for long version tampon applicator where the final tampon is sold in a deployed configuration. In this version, the user only needs to push on the proximal end of the plunger 6 in the distal direction 7 while maintaining the barrel 4 at the finger grip area 20 between the fingers. The presence of the circumferential flange 32 on the barrel 4 helps the user to better hold the barrel 4 and press the plunger 6 in the distal direction 7. Since there is only a single pushing motion, the plunger 6 can consist essentially of cellulose pulp exclusively since it doesn't require any elastic or resilient deformation.

As explained above, the barrel 4 extending in the longitudinal direction L comprises a discharge end 12 arranged at one longitudinal end, namely at its distal end, with flexible petals 16 configured to let a tampon pledget 11 pass through. The barrel 4 also comprises an open end 14 arranged at the opposite longitudinal end, namely its proximal end, with a finger grip area 20 configured to provide anti-slip texture when expelling the tampon pledget 11 and eventually a circumferential 32 at the outer edge of the proximal end. The barrel 4 also comprises a main body section 13 arranged between the discharge end 12 and the finger grip area 20. According to the invention, the petals 16 and the finger grip area 20 each consists essentially of polymeric material or a combination of cellulose pulp and polymeric material, the main body section 13 consisting essentially of cellulose pulp.

As described above, for example, the petal 16 and the finger grip area 20 can comprise a layer of cellulose pulp with a layer of bioplastic overmoulded onto the layer of cellulose pulp. Another embodiment of the tampon applicator 2 is with petals 16 made from polymeric material exclusively and the finger grip area 20 comprising a cardboard layer, in other words the continuation of the main body section 13, with the deformations 22 consisting essentially of polymeric material, e.g. polymeric ribs or rings 22 overmoulded at the proximal end of the barrel 4.

Using a 100 wt. % cellulose pulp is not desired since cardboard is relatively rigid and rough and is limited in terms of process possibilities. For example, petals 16 made from cardboard are not flexible enough and can impede the good expulsion of the tampon pledget 11. A finger grip area 20 made from cardboard is also unsatisfactory as the material is too smooth and cannot provide a desired texture to the tampon applicator 2, or in other terms, cardboard does not offer a satisfactory anti-slip feature. Thus, the tampon applicator according to the invention has the benefit of limiting the environmental impact given that cellulose pulp is a biodegradable and marine degradable material and makes up the majority of the applicator weight composition while maintaining a satisfactory level of performance in terms of expulsion and finger grip texture for example.

Of course, other elements of the tampon applicator 2 ensuring a specific function can consist essentially of polymeric material or a combination of cellulose pulp and polymeric material.

For example, the circumferential flange 32 and retention flange 34 can both consists essentially of polymeric material to help the maintaining and the pressing of the barrel 4 and plunger 6 respectively when expulsing the tampon pledget 11.

Another benefit of the invention is that it enables to expand the range of cardboard applicators. There are different types of tampon applicator, compact version, long version and a version where the tampon applicator comprises a plunger in two pieces. Standard cardboard applicators sold on the market are long applicators, meaning that the tampon applicator is ready to use, i.e. the plunger is already in a deployed configuration and a user just has to push the plunger 6 in the distal direction 7 as explained above. Standard cardboard applicators cannot be compact because the plunger 6 and the barrel 4 must present specific features with elastic deformations and retention means which is not possible for cardboard.

In the compact version, the tampon assembly 1 is sold in a compact configuration as illustrated in FIG.1, where the tampon pledget 11 is partially arranged within both plunger 6 and barrel 4 and the plunger 6 is mostly arranged within the barrel 4. The user has to pull the plunger 6 in the proximal direction 9 to reach the deployed configuration and then push again on the plunger 6 in the distal direction 7 so that the tampon pledget 11 can be expelled from the barrel 4 through the discharge end 12.

Although not illustrated in the figures, the present compact tampon applicators preferably include a restraining means, e.g., an interlocking restraining means, to prevent disassembly of the barrel 4 and plunger 6 tubes during the pulling or cocking step. Typical restraining means may be formed by circumferentially extending, raised structures (such as, e.g., ring, ribs, rims, flanges, lips or other-shape protrusions) provided on the outer distal surface of the plunger 6 and on the inner proximal surface of the barrel 4. For example, a stopping ring may project radially inwardly from the inner surface of the barrel 4 near its proximal end and another ring or a plurality of circumferentially aligned protrusions may project radially outwardly from the outer surface of the plunger 6 near its distal end (usually disposed proximally adjacent to the base of the fingers 40 of the plunger 6 that will be described afterward). The height of these raised structures is such that they are radially spaced from the opposing surface of the tube other than that on which they are provided when the plunger 6 is inserted in the barrel 4 and when it is being withdrawn in the proximal direction most of the way. However, the structures overlap radially, and will eventually engage when the plunger 6 is sufficiently withdrawn from the barrel 4, thus preventing complete withdrawal and disassembly of the applicator. These restraining means must have a certain rigidity to ensure that the plunger 6 stays at least partially within the barrel 4 and cardboard is not rigid enough. According to an aspect of the invention, these restraining means consist essentially of polymeric material whereas the rest of the plunger and/or the main body section 13 of the barrel consists essentially of cellulose pulp.

According to another aspect, compact tampon applicators commonly include retaining means 38 configured to secure the stored tampon pledget 11 in the distal end of the barrel 4. As exemplified in the figures, such retaining means 38 may typically include a plurality of, e.g., 2 to 16, more commonly 2 to 8, and most commonly 4 inward protrusions 38 on the inner surface of the outer tube 4, circumferentially arranged approximately at the base of the petals 16. The retaining means 38 may alternatively be formed as a continuous raised ring approximately at the base of the petal 16 sections, instead of a plurality of inward protrusions 38.

The inward protrusions 38 are configured to engage the distal end or insertion end of the tampon pledget 11 stored in the distal end of the barrel 4, the tampon pledget 11 being also stored mostly within the plunger 6, thereby preventing axial displacement of the tampon pledget 11 in the proximal direction 9 during the cocking or pulling of the plunger 6. The protrusions 38 may have various shapes known in the art, such as, e.g., pins, ribs, lips or flaps protruding substantially radially inwardly from the inner surface of the barrel 4, and preferably slanted towards the distal discharge end 12 thereof. The slanting of the protrusions 38 towards the distal end 3 of the barrel 4 facilitates a secure grip of the tampon pledget 11 while not interfering or interfering only minimally with the ejection of the tampon pledget 11.

The particular retaining means 38 for securing the tampon pledget 11 exemplified in the figures include eight protrusions 38, two disposed at the base of each petal section 6. They display a substantially right triangular profile with the hypotenuse (i.e., the longest side) extending at an acute angle from the inner wall of the outer tube 4 towards the central opening at the distal end 3, one of the catheti (i.e., the shorter sides) facing the distal end 3 of the barrel 4, and the other cathetus being the contact side with the inner surface of the barrel 4. The illustrated projections are relatively thin in the circumferential direction, with thickness ranging, e.g., from 0.2 mm to 5 mm, thus saving raw material. Again these retaining means 38 must have a certain rigidity to ensure that the tampon pledget 11 stays within the barrel 4 while the plunger 6 is being pulled in the proximal direction 9 and cardboard is not rigid enough. Also, it is complicated to implement such protrusions 38 with cardboard given that cardboard is not a material as malleable as polymer. According to an aspect of the invention, these retaining means 38 consist essentially of polymeric material whereas the rest of the main body section 13 of the barrel 4 consists essentially of cellulose pulp.

As illustrated in FIG. 5, another mean for retaining the tampon pledget within the barrel 4 when pulling on the plunger 6 is to arrange a resilient plate 42 on inner surface of the barrel 4, or in other terms to arrange a resilient tongue 42 extending inwardly in the interior cavity 10. In this embodiment, the plunger 6 comprises a slit 46 which is slightly wider than the resilient plate 42 so that the resilient plate 42 does not impede the sliding of the plunger 6. The resilient plate 42 is slanted inward so that when pulling on the plunger 6 the tampon pledget 11 course is stopped by the resilient plate 42 and stays within the barrel 4. The slit 46 can extend up to the distal end of the plunger 6 or can extend to a point below the base of the fingers 40 (that are described more in depth afterward) so that the fingers 40 have a higher contact surface with the proximal end of the tampon pledget 11. This retaining mean must have a certain rigidity to ensure that the tampon pledget 11 stays within the barrel 4 while the plunger 6 is being pulled in the proximal direction 9 and cardboard is not rigid enough. According to an aspect of the invention, the resilient plate 42 consists essentially of polymeric material whereas the rest of the main body section 13 of the barrel 4 consists essentially of cellulose pulp.

As explained previously, the distal end of the plunger 6 is used to push against the tampon pledget 11 and expel said pledget 11 out of the barrel 4 through the discharge end 12. To that end, the distal end of the plunger 6 of the present tampon applicator 2 is formed with one or more conventional fingers 40. Preferably, the plunger 6 may comprise a plurality, e.g., 2 to 16, preferably 2 to 8, more preferably 2 to 6, and most commonly four such fingers 40. The fingers 40 are commonly separated from one another by slots or slits, usually straight longitudinal slots or slits. The fingers 40 of the plunger 6 are made to be biased radially inwardly. Commonly, the fingers 40 may be biased such that their distal edges define an imaginary circumference smaller than the circumference defined by the base of the fingers 40 and smaller than the circumference of the tampon pledget 11 in order to push it. Hence, the fingers 40 of the plunger 6 may be radially inwardly slanted or inclined and/or may display a propensity to slant or incline radially inwardly. The fingers 40 are preferably substantially elastically flexible or bendable, to allow for their opening out when pushed radially outwardly, e.g., when plunger 6 accommodates a tampon pledget 11 or when the plunger 6 is being slid over the tampon pledget 11 surface during the cocking step, i.e. pulling in the proximal direction 9 step. However, one shall appreciate that the fingers 40 need to be sufficiently firm to adequately engage with the base of the tampon pledget 11 during the ejection step. Again these fingers 40 must have a certain flexibility to ensure that the tampon pledget 11 can deform the finger 40 while the plunger 6 is being pulled in the proximal direction 9. These fingers 40 must also have a certain rigidity to ensure that the distal end of the plunger 6 can effectively push the tampon pledget 11 when the plunger 6 is being pushed in the distal direction 7. Cardboard is neither flexid nor rigid enough. According to an aspect of the invention, these fingers 40 consist essentially of polymeric material whereas the rest of the plunger 6 consists essentially of cellulose pulp.

When a tampon applicator assembly 1 comprising a tampon applicator 2 as taught herein and a tampon pledget 11 is provided to a consumer for use, the plunger 6 is for its most part disposed within the barrel 4 and over a stored tampon pledget 11. Generally, the plunger 6 may be telescoped into the barrel 4 sufficiently far to minimise the overall length of the tampon applicator assembly 1, but not so far as to hamper grasping of the proximal end of the plunger 6 by the user or to cause the plunger 6 and/or the resident tampon pledget 11 to push open the petals 16 of the barrel 4. The distal edges of the fingers 40 of the plunger 6 may axially ("axially" is used throughout in connection to the longitudinal axis L of the tampon applicator 2) abut the inward protrusions 38 of the barrel 4 configured to engage and secure the tampon pledget 11, but commonly the fingers 40 do not substantially axially overlap with the inward protrusions 8. Alternatively, the inward protrusions 38 may project through the slots separating the fingers 40 of the plunger 6 to engage the tampon pledget 11. The fingers 40 of the plunger 6 may be forced radially outward by the stored tampon pledget 11. This permits the fingers 40, and the remainder of the plunger 6, to pass over the tampon pledget 11 that is secured in the barrel 4 by the inward projections 38 during the cocking or pulling of the plunger 6. Then, because of the radially inward bias of the fingers 40, when the plunger 6 is sufficiently withdrawn, the fingers 40 will incline or flex inwardly to a lesser diameter than that of the base of the tampon. Consequently, when the plunger 6 is axially moved in the distal direction 7, the tampon pledget 11 will be engaged at its proximal end by the fingers 40 and urged out the distal discharge end 12 of the barrel 4.

The invention is not limited to one particular embodiment. It is possible to combine several of the embodiments described above. The barrel 4 can comprise several discrete and/or separate elements consisting essentially of polymeric material or a combination of polymeric material and cellulose pulp, such as the petals 16, the finger grip area 20, the circumferential flange 32, the resilient plate 42 and/or the retaining means 38, the rest of the barrel 4, namely the main body section 13 consisting essentially of cellulose pulp. The plunger 6 can comprise several discrete and/or separate elements consisting essentially of polymeric material or a combination of polymeric material and cellulose pulp, such as the fingers 40 and/or the retention flange 34, the rest of the plunger 6 consisting essentially of cellulose pulp.

According to an embodiment, the tampon applicator 2 comprises at least two discrete areas comprising polymeric material, the polymeric material in one area being different from the polymeric material in another area. We have seen that several polymeric materials and bioplastics or a mixture of both can be used. According to an embodiment, a first polymeric material is used for the petals 16 which requires foremost flexibility and a second polymeric material is used for the retaining means 38 which requires foremost rigidity, the first and second polymeric material being of different composition, formula, and/or density. It is possible to use a third, or more, different polymeric material for other elements of the tampon applicator 2. It is possible to use a polymeric material with the most well-adapted properties for a specific element of the tampon applicator that has a function that requires certain properties for example and not limited to flexibility, rigidity, texture.

As explained above, a tampon applicator 2 can also have a plunger 6 that is in two pieces. In this version the tampon applicator 2, comprises a barrel 4 as described above and a telescoping two-piece plunger 6 that includes an outer sleeve 6a and an inner sleeve 6b and both may have a flared portion. The plunger 6 is configured to selectively reside in a compact configuration (as illustrated in FIG.6) or in a deployed configuration. In the compact configuration substantially all of the inner sleeve 6b is disposed within the outer sleeve 6a and substantially all of the outer sleeve 6a is disposed within the interior cavity 10 of the barrel 4, and in the deployed configuration, substantially all of the inner sleeve 6b is disposed outside the outer sleeve 6a. According to the invention, the outer sleeve 6a and/or inner sleeve 6b can comprise an area or an element consisting essentially of polymeric material and the rest of each sleeve 6a,6b can consist essentially of cellulose pulp. For example, the inner sleeve 6b can comprise ribs or grooves at its distal end whereas the outer sleeve 6a can comprise corresponding, or complementary, grooves or ribs at its proximal end. The inner sleeve 6b is pulled in the proximal direction 9 during the cocking step, until the ribs or grooves on the inner sleeve 6b interlock with the grooves or ribs on the outer sleeve 6a. The inner sleeve 6b can then be pushed in the proximal direction 7 so that the outer sleeve 6a can push the tampon pledget 11 through the discharge end 12. The ribs or grooves, or more broadly the interlocking means, of the outer sleeve 6a and/or inner sleeve 6b can consist essentially of polymeric material or a combination of polymeric material and cardboard whereas the rest of the outer sleeve 6a and/or inner sleeve 6b consists essentially of cellulose pulp. Given that the tampon pledget 11 is not stored within the plunger 6 in this embodiment, this embodiment offers an alternative to the retaining means 38 and resilient plate 42 described above.

In order to manufacture such tampon applicator, it is preferable to use a 2K molding process. For example, publication US 2015/0336311 A1 describes a process whereby reducing the melt pressure to 25 MPa, it is possible to overmould polymeric material onto fragile hollow items such as a cardboard barrel 4 or cardboard plunger 6. Paragraphs [0072], [0073], [0074], [0075] of said publication describing the mold and paragraphs [0082], [0083], [0084], [0085] and [0092] of said publication describing the process steps are all incorporated by reference in this description, as well as the drawings illustrating these paragraphs. Paragraphs [0086], [0087], [0088], [0089], [0090] and [0091] describing the controlling system of the process is also incorporated by reference in this description, as well as the drawings illustrating these paragraphs.

The invention also pertains to a process, more specifically an injection moulding process, for manufacturing a tampon applicator 2 as described above comprising the steps:
- providing a cardboard shell in a mould comprising at least the main body section 13 of the barrel 4 or the main body of the plunger 6;
- injecting the polymeric material in the targeted pre-defined areas;
- removing the tampon applicator 2 from the mould.

It is possible to repeat these steps as many times as needed.

For example, the process for manufacturing a tampon applicator 2 can comprise the following steps:
- providing in a mould a cardboard shell comprising at least the main body section 13 of the barrel 4;
- injecting the polymeric material on the cardboard petals 16;
- removing the tampon applicator 2 from the mould;
- vertically rotating by 180° the cardboard shell;
- providing same cardboard shell in the mould;
- injecting the polymeric material on the finger grip area 20 in circumferential rings;
- removing the tampon applicator 2 from the mould.

To manufacture a tampon applicator 2 comprising retraining means 38 or a resilient plate 42, the process comprises the following additional steps:
- punching at least one hole in the barrel 4,
- injecting polymeric material in said at least one hole.

It is supposed that the present invention is not restricted to any form of realization described previously and that some modifications can be added to the presented example of fabrication without reappraisal of the appended claims.

## Claims

1. Tampon applicator (2) wherein the tampon applicator (2) comprises cellulose pulp and discrete areas of polymeric material.

2. Tampon applicator (2) according to claim 1, wherein the tampon applicator (2) comprises between about 75 % to about 97 % in weight of cellulose pulp and between about 3 % to about 25 % in weight of polymeric material.

3. Tampon applicator (2) according to any of the preceding claims, wherein the polymeric material consists essentially of a bioplastic derived from biomass and/or a compostable polymeric material and/or a water-soluble polymeric material and/or a marine biodegradable polymeric material.

4. Tampon applicator (2) according to any of the preceding claims, the tampon applicator (2) comprising:
- a barrel (4) defining an interior cavity (10),
- a plunger (6) arranged to slide within the barrel (4),
wherein the barrel (4) comprises cellulose pulp and discrete areas of polymeric material.

5. Tampon applicator (2) according to any of the claims 1 to 3, the tampon applicator (2) comprising:
- a barrel (4) defining an interior cavity (10),
- a plunger (6) arranged to slide within the barrel (4),
wherein the plunger (6) comprises cellulose pulp and discrete areas of polymeric material.

6. Tampon applicator (2) according to claim 4 or 5, the barrel (4) extending in a longitudinal direction (L) further comprising:
- a discharge end (12) arranged at a first longitudinal end, or distal end, of the barrel (4) and comprising flexible petals (16),
- a finger grip area (20) arranged at the opposite longitudinal end, or proximal end, of the barrel (4),
- a main body section (13) arranged between the discharge end (12) and the finger grip area (20),
wherein the petals (16) and the finger grip area (20) consists essentially of polymeric material or a combination of cellulose pulp and polymeric material and the main body section (13) consists essentially of cellulose pulp.

7. Tampon applicator (2) according to any of the claims 4, 5 or 6, the barrel (4) further comprising at its distal end, tampon pledget (11) retaining means (38) comprising a plurality of protrusions (38) extending inward in the interior of the barrel (4), wherein the retaining means (38) consist essentially of polymeric material.

8. Tampon applicator according to any of the claims 5 to 7, wherein the plunger (6) comprises at least one finger (40) at its distal end, wherein the at least one finger (40) consists essentially of polymeric material.

9. Tampon applicator according to any of the claims 4 to 8, wherein the barrel (4) comprises at its proximal end a circumferential flange (32) extending outward and/or the plunger (6) comprises at its proximal end a retention flange (34) extending outward, the circumferential flange (32) and/or retention flange (34) consisting essentially of polymeric material.

10. Tampon applicator (2) according to any of the claims 4 to 9, the barrel (4) further comprising at its proximal end a tampon pledget (11) retaining mean comprising a resilient plate (42) extending in the interior of the barrel (4), the plunger (6) comprising a slit (46) extending in the longitudinal direction of the plunger (6), the resilient plate (42) being arranged within the slit (46), wherein the resilient plate (42) consists essentially of polymeric material and/or cellulose pulp.

11. Tampon applicator according to any of the claims 4 to 10, wherein the barrel (4) and/or plunger (6) comprise at least two discrete areas comprising polymeric material, the polymeric material in first area being different from the polymeric material in another area.

12. Tampon applicator (2) according to any of the claims 4 to 11, wherein, the plunger (6) comprises an inner sleeve (6b) and an outer sleeve (6a) with interlocking means, said inner sleeve (6b) and/or outer sleeve (6a) comprising at least one discrete area of polymeric material.

13. Process for manufacturing a tampon applicator according to claim 4 to 12 comprising the steps:
- providing a cardboard shell comprising at least the main body section (13) of the barrel (4) or the main body of the plunger (6);
- injecting the polymeric material in the targeted pre-defined areas;
- removing the tampon applicator (2).

14. Process according to claim 13 for manufacturing a tampon applicator according to claim 7 or 10 and depending claims, wherein the process comprises the following additional steps:
- Punching holes in the carboard shell,
- injecting polymeric material in said holes.

## Patentansprüche

1. Tamponapplikator (2), wobei der Tamponapplikator (2) Zellstoff und diskrete Bereiche aus Polymermaterial umfasst.

2. Tamponapplikator (2) nach Anspruch 1, wobei der Tamponapplikator (2) zwischen etwa 75 Gew.-% bis etwa 97 Gew.-% Zellstoff und zwischen etwa 3 Gew.-% bis etwa 25 Gew.-% Polymermaterial umfasst.

3. Tamponapplikator (2) nach einem der vorstehenden Ansprüche, wobei das Polymermaterial im Wesentlichen aus einem aus Biomasse abgeleiteten Biokunststoff und/oder einem kompostierbaren Polymermaterial und/oder einem wasserlöslichen Polymermaterial und/oder einem im Meer biologisch abbaubaren Polymermaterial besteht.

4. Tamponapplikator (2) nach einem der vorstehenden Ansprüche, wobei der Tamponapplikator (2) umfasst:
- einen Zylinder (4), der einen Innenhohlraum (10) definiert,
- einen Kolben (6), der angeordnet ist, um innerhalb des Zylinders (4) zu gleiten, wobei der Zylinder (4) Zellstoff und diskrete Bereiche aus Polymermaterial umfasst.

5. Tamponapplikator (2) nach einem der Ansprüche 1 bis 3, wobei der Tamponapplikator (2) umfasst:
- einen Zylinder (4), der einen Innenhohlraum (10) definiert,
- einen Kolben (6), der angeordnet ist, um innerhalb des Kolbens (4) zu gleiten, wobei der Kolben (6) Zellstoff und diskrete Bereiche aus Polymermaterial umfasst.

6. Tamponapplikator (2) nach Anspruch 4 oder 5, wobei der Zylinder (4), der sich in einer Längsrichtung (L) erstreckt, weiter umfasst:
- ein Austrittsende (12), das an einem ersten Längsende, oder distalen Ende, des Zylinders (4) angeordnet ist und flexible Blätter (16) umfasst,
- einen Fingergriffbereich (20), der am entgegengesetzten Längsende, oder proximalen Ende, des Zylinders (4) angeordnet ist,
- einen Hauptkörperabschnitt (13), der zwischen dem Austrittsende (12) und dem Fingergriffbereich (20) angeordnet ist,
wobei die Blätter (16) und der Fingergriffbereich (20) im Wesentlichen aus Polymermaterial oder einer Kombination aus Zellstoff und Polymermaterial bestehen, und der Hauptkörperabschnitt (13) im Wesentlichen aus Zellstoff besteht.

7. Tamponapplikator (2) nach einem der Ansprüche 4, 5 oder 6, wobei der Zylinder (4) an seinem distalen Ende weiter Haltemittel (38) für den Tamponbausch (11) umfasst, die eine Vielzahl von Vorsprüngen (38) umfassen, die sich nach innen in das Innere des Zylinders (4) erstrecken, wobei die Haltemittel (38) im Wesentlichen aus Polymermaterial bestehen.

8. Tamponapplikator nach einem der Ansprüche 5 bis 7, wobei der Kolben (6) an seinem distalen Ende mindestens einen Finger (40) umfasst, wobei der mindestens eine Finger (40) im Wesentlichen aus Polymermaterial besteht.

9. Tamponapplikator nach einem der Ansprüche 4 bis 8, wobei der Zylinder (4) an seinem proximalen Ende einen Umfangsflansch (32) umfasst, der sich nach außen erstreckt, und/oder der Kolben (6) an seinem proximalen Ende einen Rückhalteflansch (34) umfasst, der sich nach außen erstreckt, wobei der Umfangsflansch (32) und/oder der Rückhalteflansch (34) im Wesentlichen aus Polymermaterial bestehen.

10. Tamponapplikator (2) nach einem der Ansprüche 4 bis 9, wobei der Zylinder (4) an seinem proximalen Ende weiter ein Haltemittel für den Tamponbausch (11) umfasst, das eine elastische Platte (42) umfasst, die sich im Inneren des Zylinders (4) erstreckt, wobei der Kolben (6) einen Schlitz (46) umfasst, der sich in Längsrichtung des Kolbens (6) erstreckt, wobei die elastische Platte (42) innerhalb des Schlitzes (46) angeordnet ist, wobei die elastische Platte (42) im Wesentlichen aus Polymermaterial und/oder Zellstoff besteht.

11. Tamponapplikator nach einem der Ansprüche 4 bis 10, wobei der Zylinder (4) und/oder der Kolben (6) mindestens zwei diskrete Bereiche umfassen, die Polymermaterial umfassen, wobei sich das Polymermaterial im ersten Bereich vom Polymermaterial in einem anderen Bereich unterscheidet.

12. Tamponapplikator (2) nach einem der Ansprüche 4 bis 11, wobei der Kolben (6) eine innere Hülse (6b) und eine äußere Hülse (6a) mit Verriegelungsmitteln umfasst, wobei die innere Hülse (6b) und/oder die äußere Hülse (6a) mindestens einen diskreten Bereich aus Polymermaterial umfasst.

13. Prozess zum Herstellen eines Tamponapplikators nach Anspruch 4 bis 12, der die Schritte umfasst:
- Bereitstellen einer Kartonhülle, die mindestens den Hauptkörperabschnitt (13) des Zylinders (4) oder den Hauptkörper des Kolbens (6) umfasst;
- Einspritzen des Polymermaterials in die vordefinierten Zielbereiche;
- Entfernen des Tamponapplikators (2).

14. Prozess nach Anspruch 13 zum Herstellen eines Tamponapplikators nach Anspruch 7 oder 10 und abhängigen Ansprüchen, wobei der Prozess die folgenden zusätzlichen Schritte umfasst:
- Stanzen von Löchern in die Kartonhülle,
- Einspritzen von Polymermaterial in die Löcher.

## Revendications

1. Applicateur (2) de tampon, dans lequel l'applicateur (2) de tampon comprend de la pâte de cellulose et des zones distinctes de matériau polymère.

2. Applicateur (2) de tampon selon la revendication 1, dans lequel l'applicateur (2) de tampon comprend d'environ 75 % à environ 97 % en poids de pâte de cellulose et d'environ 3 % à environ 25 % en poids de matériau polymère.

3. Applicateur (2) de tampon selon l'une quelconque des revendications précédentes, dans lequel le matériau polymère consiste essentiellement en un bioplastique dérivé de biomasse et/ou un matériau polymère compostable et/ou un matériau polymère hydrosoluble et/ou un matériau polymère biodégradable dans l'eau de mer.

4. Applicateur (2) de tampon selon l'une quelconque des revendications précédentes, l'applicateur (2) de tampon comprenant :
- un cylindre (4) définissant une cavité intérieure (10),
- un piston (6) conçu pour glisser à l'intérieur du cylindre (4), dans lequel le cylindre (4) comprend de la pâte de cellulose et des zones distinctes de matériau polymère.

5. Applicateur (2) de tampon selon l'une quelconque des revendications 1 à 3, l'applicateur (2) de tampon comprenant :
- un cylindre (4) définissant une cavité intérieure (10),
- un piston (6) conçu pour glisser à l'intérieur du cylindre (4), dans lequel le piston (6) comprend de la pâte de cellulose et des zones distinctes de matériau polymère.

6. Applicateur (2) de tampon selon la revendication 4 ou 5, le cylindre (4) s'étendant selon une direction longitudinale (L) comprenant en outre :
- une extrémité d'évacuation (12) agencée au niveau d'une première extrémité longitudinale, ou extrémité distale, du cylindre (4) et comprenant des pétales flexibles (16),
- une zone de prise (20) pour les doigts agencée au niveau de l'extrémité longitudinale opposée, ou extrémité proximale, du cylindre (4),
- une section corps principal (13) agencée entre l'extrémité d'évacuation (12) et la zone de prise (20) pour les doigts,
dans lequel les pétales (16) et la zone de prise (20) pour les doigts consistent essentiellement en un matériau polymère ou une combinaison de pâte de cellulose et de matériau polymère et la section corps principal (13) consiste essentiellement en de la pâte de cellulose.

7. Applicateur (2) de tampon selon l'une quelconque des revendications 4, 5 ou 6, le cylindre (4) comprenant en outre au niveau de son extrémité distale, des moyens de retenue (38) de compresse (11) de tampon comprenant une pluralité de saillies (38) s'étendant vers l'intérieur dans l'intérieur du cylindre (4), dans lequel les moyens de retenue (38) consistent essentiellement en un matériau polymère.

8. Applicateur de tampon selon l'une quelconque des revendications 5 à 7, dans lequel le piston (6) comprend au moins un doigt (40) au niveau de son extrémité distale, dans lequel le au moins un doigt (40) consiste essentiellement en un matériau polymère.

9. Applicateur de tampon selon l'une quelconque des revendications 4 à 8, dans lequel le cylindre (4) comprend au niveau de son extrémité proximale une bride circonférentielle (32) s'étendant vers l'extérieur et/ou le piston (6) comprend au niveau de son extrémité proximale une bride de retenue (34) s'étendant vers l'extérieur, la bride circonférentielle (32) et/ou la bride de retenue (34) consistant essentiellement en un matériau polymère.

10. Applicateur (2) de tampon selon l'une quelconque des revendications 4 à 9, le cylindre (4) comprenant en outre au niveau de son extrémité proximale un moyen de retenue de compresse (11) de tampon comprenant une plaque souple (42) s'étendant à l'intérieur du cylindre (4), le piston (6) comprenant une fente (46) s'étendant dans la direction longitudinale du piston (6), la plaque souple (42) étant agencée à l'intérieur de la fente (46), dans lequel la plaque souple (42) consiste essentiellement en un matériau polymère et/ou de la pâte de cellulose.

11. Applicateur de tampon selon l'une quelconque des revendications 4 à 10, dans lequel le cylindre (4) et/ou le piston (6) comprend au moins deux zones distinctes comprenant un matériau polymère, le matériau polymère dans la première zone étant différent du matériau polymère dans une autre zone.

12. Applicateur (2) de tampon selon l'une quelconque des revendications 4 à 11, dans lequel le piston (6) comprend un manchon interne (6b) et un manchon externe (6a) avec des moyens d'enclenchement, ledit manchon interne (6b) et/ou ledit manchon externe (6a) comprenant au moins une zone distincte de matériau polymère.

13. Processus de fabrication d'un applicateur de tampon selon les revendications 4 à 12 comprenant les étapes de :
- fourniture d'une coque en carton comprenant au moins la section corps principal (13) du cylindre (4) ou le corps principal du piston (6) ;
- injection du matériau polymère dans les zones prédéfinies ciblées ;
- retrait de l'applicateur (2) de tampon.

14. Processus selon la revendication 13 de fabrication d'un applicateur de tampon selon la revendication 7 ou 10 et les revendications dépendantes, dans lequel le processus comprend les étapes supplémentaires suivantes de :
- perçage de trous dans la coque en carton,
- injection du matériau polymère dans lesdits trous.
